# EUROPEAN PATENT SPECIFICATION

(11) **EP 0 462 514 B1**
(45) Date of publication and mention of the grant of the patent: **07.09.1994**
(21) Application number: 91109749.1
(22) Date of filing: 14.06.1991
(51) Int. Cl.: C07C 19/08, C07C 17/00

(54) **Process for preparing 1-chloro-2,2,2-trifluoroethane**
Verfahren zur Herstellung von 1-Chlor-2,2,2-trifluorethan
Procédé pour la préparation de 1-chloro-2,2,2-trifluoroéthane

(30) Priority: 18.06.1990 JP 158962/90
(43) Date of publication of application: 27.12.1991
(73) Proprietor: DAIKIN INDUSTRIES, LIMITED, Osaka-shi, Osaka-fu 530 (JP)
(72) Inventor: Nakada, Tatsuo, Yodogawa Works of Daikin Ind. Ltd., Settsu-shi, Osaka-fu (JP); Koyama, Satoshi, Yodogawa Works of Daikin Ind.Ltd., Settsu-shi, Osaka-fu (JP)
(74) Representative: Hansen, Bernd, Dr. Dipl.-Chem.

(56) References cited:
- FR-A- 2 388 785
- US-A- 3 003 003

## Description

### BACKGROUND OF THE INVENTION

### Field of the Invention

The present invention relates to a process for preparing 1-chloro-2,2,2-trifluoroethane (hereinafter referred to as R-133a). R-133a as such is useful as an intermediate in the preparation of a pharmaceutical or agricultural compound, and is fluorinated with anhydrous hydrogen fluoride to give to tetrafluoroethane which acts as a working fluid.

### Description of the Related Art

It is known that R-133a is synthesized by contacting trichloroethylene and hydrogen fluoride (HF) to a catalyst compound of the formula:

SbClₓF_{y}

wherein x and y are both a positive number and the sum of x and y is 5. However, by this synthesis process, a large amount of chlorides are by-produced and Sb in the catalyst is deteriorated to a trivalent metal. To reactivate the deteriorated catalyst, chlorine (Cl₂) is introduced in a reaction system. Therefore, both a yield and a selectivity are very low, and this process is not an industrial process which can produce R-133a easily at a low cost (see U.S. Patent No. 3,003,003).

In a conventional process disclosed in Japanese Patent Kokai Publication No. 135909/1978, a large excess amount of HF is used in the synthesis of R-133a. However, only about 50 % of HF is utilized. When an exceeding amount of HF is diminished in this process, the selectivity greatly decreases. Since this process is a batchwise process so that an operating efficiency of an apparatus is low when it is carried out in an industrial scale, it is not easily operable process.

FR-2 388 785 discloses a process for the preparation of 1,1,1-trifluoro-2-chloroethane by reacting trichloroethylene with hydrogen fluoride in the presence of a catalyst consisting of one or more compounds of antimony and/or arsenic and one or more compounds including a transition metal. According to a preferred embodiment, the transition metal is represented by zinc, cadmium, mercury, nickel, cobalt and iron. A specific preferred embodiment of the catalyst is represented by a combination of antimony pentachloride and a compound of zinc, cadmium and/or mercury.

### SUMMARY OF THE PRESENT INVENTION

One object of the present invention is to provide a process for preparing R-133a in a good yield and selectivity.

Another object of the present invention is to provide a process for preparing R-133a economically in an industrial scale.

According to the present invention, there is provided a process for preparing R-133a comprising fluorinating trichloroethylene with hydrogen fluoride in the presence of a catalyst consisting of a compound of the formula:

SbClₓF_{y}

wherein x and y are both a positive number and the sum of x and y is 5, wherein the amount of hydrogen fluoride in the reaction system is at least five moles per one mole of the catalyst.

### DETAILED DESCRIPTION OF THE INVENTION

In a preferred embodiment, the fluorination is carried out by introducing trichloroethylene and hydrogen fluoride in the reaction system and recovering R-133a from the reaction system while keeping a reaction pressure constant.

Preferably, a molar ratio of HF to trichloroethylene is at least 4:1, and prepared R-133a is recovered from the reaction system as an azeotropic mixture with HF.

While trichloroethylene is cheap and desirable as a starting material, in addition to or in place of trichloroethylene, 1,2,2-trichloro-2-fluoroethane or 1,2-dichloro-2,2-difluoroethane may be used as a starting material. 1,2,2-Trichloro-2-fluoroethane or 1,2-dichloro-2,2-difluoroethane is an intermediate of the above fluorination reaction, and it does not interfere with the fluorination reaction. Since the addition reaction of HF to trichloro-ethylene and fluorination of 1,2,2-trichloro-2-fluoroethane or 1,2-dichloro-2,2-difluoroethane proceed successively, they may be carried out in series.

An amount of HF is usually from 5 to 500 moles, preferably from 50 to 300 moles per one mole of the SbClₓF_{y} catalyst. When the amount of HF is larger than the above upper limit, a productivity per a unit volume of a reactor decreases, although the reaction itself is not influenced. When the amount of HF is smaller than the above lower limit, though the reaction proceeds, an amount of trichloroethylene to be introduced should be decreased so as to avoid decrease of the selectivity, and efficiency is deteriorated.

The amount of HF is at least a total amount of HF which is consumed in the reaction and HF which forms the azeotropic mixture. When the amount of introduced HF is too large, the productivity of R-133a undesirably decreases, and a utilization factor of HF decreases, since the amount of HF in the reaction system is kept constant. Preferably, the amount of introduced HF is from 4 to 8 moles per one mole of trichloroethylene, from 3 to 6 moles per one mole of 1,2,2-trichloro-2-fluoroethane, or from 2 to 4 moles per one mole of 1,2-dichloro-2,2-difluoroethane.

An amount of trichloroethylene to be introduced in the reactor is 5 to 100 mol/hour, preferably from 10 to 50 mol/hour per one mole of the SbClₓF_{y} catalyst. When the amount of trichloroethylene is too small, the productivity decreases although the reaction proceeds. When the amount of trichloroethylene is too large, the content of fluorine in the SbClₓF_{y} catalyst decreases so that the selectivity decreases though the reaction proceeds.

The reaction is carried out at a temperature of 30°C or higher. At a temperature slightly higher than 30°C, the selectivity decreases if the amount of introduced trichloroethylene is not small in relation to the amount of the SbClₓF_{y} catalyst. Increase of the reaction temperature may be favorable for the productivity and the selectivity, but at high reaction temperature, a reaction pressure should be kept high. Since a high reaction pressure increases the cost of an equipment, practically the reaction temperature is from 50 to 150°C.

The reaction pressures is selected from a range between 3 and 30 kg/cm² so as to separate R-133a from HF by increasing the pressure as the temperature is raised. It is possible to accumulate R-133a in the reaction system by the increase of reaction pressure while removing by-produced HCl from the reaction system.

To have the SbClₓF_{y} catalyst present in the reaction system, it is preferred to add antimony pentachloride to the reaction system.

It is known that added antimony pentachloride is partly fluorinated with HF to form SbClₓF_{y}. When SbClₓF_{y} is used as a catalyst for the fluorination of a compound having a hydrogen atom which can be chlorinated or a double bond such as trichloroethylene, the fluorination reaction rate increases as the content of fluorine in SbClₓF_{y} increases, whereby side reactions are suppressed.

By using the excess amount of HF in relation to antimony pentachloride to keep the fluorine content in SbClₓF_{y} high, an addition reaction is promoted and in turn R-133a is produced with a good selectivity. That is, by regenerating consumed SbClₓF_{y} having the high fluorine content with the excess amount of HF and supplying trichloroethylene at a rate which does not exceed a regeneration rate of the catalyst, the fluorine content in SbClₓF_{y} is kept high and the production of R-122, which tends to be formed when the chlorine content in SbClₓF_{y} is high, is suppressed.

By the presence of excessive HF, the addition reaction of HF to the olefin, which is a competitive reaction with chlorination, proceeds quickly.

By the process of the present invention, R-133a is prepared with a good selectivity and deterioration of SbClₓF_{y} caused by chlorination can be suppressed.

### PREFERRED EMBODIMENTS OF THE INVENTION

The present invention will be illustrated by the following Examples.

### Example 1

In a 500 ml autoclave, SbCl₅ (10 ml, 0.05 mol) was charged and cooled with dry ice, and then HF (100 ml) was introduced. After an internal temperature of the autoclave rose to room temperature, the mixture was heated at 50°C for 2 hours and then at 60°C for 6 hours, while vigorously stirring. Thereafter, in the mixture, trichloroethylene and HF were introduced at rates of 0.25 mol/hour and 1 mol/hour, respectively. The product was withdrawn from the autoclave through a cooling tube to keep the reaction pressure at 6.2 kg/cm². After carrying out the reaction for 8 hours and when 2 moles of trichloroethylene were supplied, the reaction was terminated, and organic materials collected in a dry ice-cooled trap were analyzed. The results are as follows:
Yield: 180 g

| GLC analysis (TCD): | |
|---|---|
| R-133a: | 93.1 % |
| R-132b (1_{,}2-dichloro-1,1-difluoroethane): | 3.4 % |
| R-122 (1,2,2-trichloro-1,1-difluoroethane): | 3.3 % |

The organic materials in the autoclave were also analyzed to find that the total yield of R-133a was 92 %.

### Example 2

In a 500 ml autoclave, SbCl₅ (10 ml, 0.05 mol) was charged and cooled with dry ice, and then HF (100 ml) was introduced. After an internal temperature of the autoclave rose to room temperature, the mixture was heated at 50°C for 2 hours and then at 60°C for 6 hours, while vigorously stirring. Thereafter, the internal temperature was further raised to 80°C and, in the mixture, trichloroethylene and HF were introduced at rates of 0.5 mol/hour and 2 mol/hour, respectively. The product was withdrawn from the autoclave through a cooling tube to keep the reaction pressure at 8.5 kg/cm². After carrying out the reaction for 4 hours and when 2 moles of trichloroethylene were supplied, the reaction was terminated, and organic materials collected in a dry ice-cooled trap were analyzed. The results are as follows:
Yield: 194 g

| GLC analysis (TCD): | |
|---|---|
| R-133a: | 97.5 % |
| R-132b: | 2.4 % |
| R-122: | 0.5 % |

The organic materials in the autoclave were also analyzed to find that the total yield of R-133a was 96 %.

### Example 3

In a 500 ml autoclave, SbCl₅ (10 ml, 0.05 mol) was charged and cooled with dry ice, and then HF (200 ml) was introduced. After an internal temperature of the autoclave rose to room temperature, the mixture was heated at 50°C for 2 hours and then at 60°C for 6 hours, while vigorously stirring. Thereafter, the internal temperature was further raised to 80°C and, in the mixture, trichloroethylene and HF were introduced at rates of 0.5 mol/hour and 2 mol/hour, respectively. To keep the reaction pressure at 8.5 kg/cm², the autoclave was cooled by circulating a cooling water of 60°C and the product was withdrawn from the autoclave through a cooling tube. After carrying out the reaction for 4 hours and when 2 moles of trichloroethylene were supplied, the reaction was terminated, and organic materials collected in a dry ice-cooled trap were analyzed. The results are as follows:
Yield: 207 g

| GLC analysis (TCD): | |
|---|---|
| R-133a: | 97.6 % |
| R-132b: | 2.3 % |
| R-122: | <0.1 % |

The organic materials in the autoclave were also analyzed to find that the total yield of R-133a was 97 %.

### Example 4

In the same manner as in Example 3 but circulating a cooling water of 40°C, the reaction was carried out. The results are as follows:
Yield: 185 g

| GLC analysis (TCD): | |
|---|---|
| R-133a: | 99.8 % |
| R-132b: | 0.2 % |

The organic materials in the autoclave were also analyzed to find that the total yield of R-133a was 97 %.

As seen from the results of Example 4, the purity of the product depends on an efficiency of the cooling tube and the number of plates. Therefore, contamination of the product with the intermediates such as 132b can be prevented easily by the present invention.

## Claims

1. A process for preparing 1-chloro-2,2,2-trifluoroethane comprising fluorinating trichloroethylene with hydrogen fluoride in the presence of a catalyst consisting of a compound of the formula:
SbClₓF_{y}
wherein x and y are both a positive number and the sum of x and y is 5, wherein an amount of hydrogen fluoride in a reaction system is at least five moles per one mole of the catalyst.

2. The process according to claim 1, wherein trichloroethylene and hydrogen fluoride are introduced in a reaction system and 1-chloro-2,2,2-trifluoroethane is recovered from the reaction system while keeping a reaction pressure constant.

3. The process according to claim 1, wherein a molar ratio of hydrogen fluoride to trichloroethylene is at least 4:1.

4. The process according to claim 3, wherein said molar ratio of hydrogen fluoride to trichloroethylene is from 4:1 to 8:1.

5. The process according to claim 2, wherein 1-chloro-2,2,2-trifluoroethane is recovered as an azeotropic mixture with hydrogen fluoride.

6. The process according to claim 1, wherein an amount of hydrogen fluoride is from 5 to 500 moles per one mole of SbClₓF_{y}.

7. The process according to claim 1, wherein a reaction temperature is at least 30°C.

8. The process according to claim 1, wherein a reaction pressure is from 3 to 30 kg/cm².

9. The process according to claim 1, wherein antimony pentachloride is added to the reaction system.

10. The process according to claim 1, wherein 1,2,2-trichloro-2-fluoroethane or 1,2-dichloro-2,2-difluoroethane is added to the reaction system.

## Patentansprüche

1. Ein Verfahren zur Herstellung von 1-Chlor-2,2,2-trifluorethan, welches die Fluorierung von Trichlorethylen mit Fluorwasserstoff in Gegenwart eines Katalysators beinhaltet, der aus einer Verbindung der Formel
SbClₓF_{y}
besteht, wobei x und y beide eine positive Zahl sind und die Summe aus x und y 5 ist, wobei der Anteil des Fluorwasserstoffs im Reaktionssystem bei mindestens 5 Molen pro 1 Mol des Katalysators liegt.

2. Das Verfahren gemäß Anspruch 1, worin Trichlorethylen und Fluorwasserstoff in das Reaktionssystem eingeführt werden, und 1-Chlor-2,2,2-trifluorethan aus dem Reaktionssystem gewonnen wird, während der Reaktionsdruck konstant gehalten wird.

3. Das Verfahren gemäß Anspruch 1, wobei das Molverhältnis von Fluorwasserstoff zu Trichlorethylen mindestens 4:1 ist.

4. Das Verfahren nach Anspruch 3, wobei das genannte Molverhältnis von Fluorwasserstoff zu Trichlorethylen zwischen 4:1 und 8:1 liegt.

5. Das Verfahren nach Anspruch 2, wobei 1-Chlor-2,2,2-trifluorethan als azeotrope Mischung mit Fluorwasserstoff gewonnen wird.

6. Das Verfahren nach Anspruch 1, wobei der Anteil des Fluorwasserstoffs zwischen 5 und 500 Molen pro 1 Mol SbClₓF_{y} liegt.

7. Das Verfahren nach Anspruch 1, wobei die Reaktionstemperatur mindestens 30°C ist.

8. Das Verfahren nach Anspruch 1, wobei der Reaktionsdruck zwischen 3 und 30 kg/cm² liegt.

9. Das Verfahren nach Anspruch 1, wobei Antimonpentachlorid dem Reaktionssystem zugefügt wird.

10. Das Verfahren gemäß Anspruch 1, wobei 1,2,2-Trichlor-2-fluorethan oder 1,2-Dichlor-2,2-dichlorethan dem Reaktionssystem zugefügt wird.

## Revendications

1. Procédé de préparation de 1-chloro-2,2,2-trifluoroéthane comprenant la fluoration de trichloroéthylène à l'aide de fluorure d'hydrogène en présence d'un catalyseur constitué d'un composé de formule :
SbClₓF_{y}
dans laquelle x et y sont tous deux des nombres positifs et la somme de x et y vaut 5, dans lequel la quantité de fluorure d'hydrogène dans le système réactionnel est d'au moins 5 moles pour 1 mode de catalyseur.

2. Procédé selon la revendication 1, dans lequel on introduit du trichloroéthylène et du fluorure d'hydrogène dans un système réactionnel et on récupère du 1-chloro-2,2,2-trifluoroéthane du système réactionnel tout en maintenant une pression réactionnelle constante.

3. Procédé selon la revendication 1, dans lequel le rapport molaire du fluorure d'hydrogène au trichloroéthylène est d'au moins 4 : 1.

4. Procédé selon la revendication 3, dans lequel ledit rapport molaire du fluorure d'hydrogène au trichloroéthylène est compris entre 4 : 1 et 8 : 1.

5. Procédé selon la revendication 2, dans lequel on récupère le 1-chloro-2,2,2-trifluoroéthane sous forme d'un mélange azéotrope avec le fluorure d'hydrogène.

6. Procédé selon la revendication 1, dans lequel la quantité de fluorure d'hydrogène est de 5 à 500 modes pour 1 mole de SbClₓF_{y}.

7. Procédé selon la revendication 1, dans lequel la température réactionnelle est d'au moins 30°C.

8. Procédé selon la revendication 1, dans lequel la pression réactionnelle est comprise entre 3 et 30 kg/cm².

9. Procédé selon la revendication 1, dans lequel on ajoute du pentachlorure d'antimoine au système réactionnel.

10. Procédé selon la revendication 1, dans lequel on ajoute du 1,2,2-trichloro-2-fluoroéthane ou du 1,2-dichloro-2,2-difluoroéthane au système réactionnel.
